# EUROPEAN PATENT SPECIFICATION

(11) **EP 4 549 917 B1**
(45) Date of publication and mention of the grant of the patent: **02.12.2026**
(21) Application number: 24209054.6
(22) Date of filing: 25.10.2024
(51) Int. Cl.: G01N 21/78, G01N 21/84

(54) **MEASUREMENT SYSTEM**
MESSSYSTEM
SYSTÈME DE MESURE

(30) Priority: 30.10.2023 JP 2023185915
(43) Date of publication of application: 07.05.2025
(73) Proprietor: ARKRAY, Inc., Kyoto-shi, Kyoto 601-8045 (JP)
(72) Inventor: ODAGAKI, Toru, Kyoto, 602-0008 (JP); SASAKI, Hyuga, Kyoto, 602-0008 (JP)
(74) Representative: Dehns

(56) References cited:
- KR-B1- 102 297 233
- US-A1- 2014 286 550
- US-A1- 2015 233 898
- US-A1- 2017 023 542
- US-A1- 2020 124 587
- US-A1- 2022 065 795

## Description

### BACKGROUND

### Technical Field

The present invention relates to a measurement system that optically measures a measurement target.

### Related Art

A fluoroscopy detection device described in Japanese Patent Application Laid-Open (JP-A) No. 2007-315772 is equipped with an illuminance sensor of a detection section, and in particular detects errors due to external light (extraneous stray light) when a shutter of an input port or an output port is not closed.

In a system configured by an optical measurement device employing an immunochromatogram test strip to perform fluorescent immunochromatography measurement in a dark space, and in which the optical measurement device is assembled by users themselves, it is necessary to check if the device is correctly assembled or if there is extraneous stray light in a measurement area. In such cases, since it is necessary to immediately use an individually sealed ordinary test strip after being opened, it is necessary to check such errors before inserting the test strip into the optical measurement device. US 2020/124587 A1 discloses a urinalysis device for non-clinical use. The device comprises at two light sources, and a test strip holder for removably providing a test strip to the device such that the test strip is illuminated by the light sources therein. US 2014/0286550 A1 discloses a portable reader module for removably receiving a portable device and for removably receiving a cartridge comprising a test strip into an internal space. A housing of the module allows the internal space to be light shielded, while an optical path is created between the test strip and an imager on the portable device. US 2022/065795 A1 discloses a dark chamber for performing a lateral flow assay. The chamber comprises a light source and an image capturing device can be aligned with the chamber for capturing images therein. US 2015/0233898 A1 discloses a test strip module wherein a test strip positioned in a case can be anchored in position and aligned with a mating surface of a mobile computing device for obtaining an image thereof. US 2017/0023542 A1 discloses a diagnostic test reader comprising a smartphone docked to a reader for imaging test strips provided to the reader. Light for the image is provided by the flash of the smartphone. KR 102297233 B1 discloses an immunoassay device for analyzing a reactant on a test strip inserted therein, an image of the test strip is then taken by a mobile terminal.

### SUMMARY

The invention provides for a measurement system according to claim 1.

According to an exemplary embodiment of the present invention, it is possible to check an assembly error in an optical measurement device without inserting a test sample carrier such as a test strip.

### BRIEF DESCRIPTION OF THE DRAWINGS

Exemplary embodiments will be described in detail based on the following figures, wherein:
Fig. 1 is an upper perspective view of a holder serving as a first element employed in an exemplary embodiment;
Fig. 2 is a lower perspective view illustrating an enlargement of a vicinity of an insertion port of a holder;
Fig. 3 is a plan view of a test sample carrier employed in an exemplary embodiment;
Fig. 4 is an upper perspective view of a state in which a test sample carrier is mounted to a holder;
Fig. 5 is a plan view of the state in Fig. 4;
Fig. 6 is an upper perspective view from above of a casing serving as a second element employed in an exemplary embodiment;
Fig. 7 is a bottom view of a casing;
Fig. 8 is an upper perspective view of a housing of an exemplary embodiment;
Fig. 9 is an upper perspective view from above of a state in which a test sample carrier is mounted to the housing of Fig. 8;
Fig. 10 is a bottom view of a smart device employed in an exemplary embodiment;
Fig. 11 is an upper perspective view of a measurement system of an exemplary embodiment;
Fig. 12 is an upper perspective view of a state in which part of an external wall section is removed from the measurement system of Fig. 11;
Fig. 13 illustrates a cross-section taken along XIII-XIII of Fig. 12;
Fig. 14 is a functional block diagram of a measurement system of an exemplary embodiment;
Fig. 15 illustrates a block diagram of a controller;
Fig. 16 is a flowchart illustrating an overview of error detection in a measurement system of an exemplary embodiment;
Fig. 17 illustrates an example of a verification area;
Fig. 18 is a flowchart illustrating an overview of a first error detection process for detecting assembly error;
Fig. 19 illustrates positions of boundary regions and an identification region in the verification area;
Fig. 20 is a flowchart illustrating an overview of a second error detection process for detecting whether or not a first element is suitable;
Fig. 21 illustrates a specific region for displaying identification information in a verification area;
Fig. 22 is a flowchart illustrating an overview of a third error detection process for detecting a filter error;
Fig. 23 illustrates a position of a measurement region in a verification area;
Fig. 24 illustrates a check region and a permitted region in a verification image captured in a dark chamber; and
Fig. 25 is a flowchart illustrating an overview of a fourth error detection process for detecting stray light error.

### DETAILED DESCRIPTION

Description follows regarding exemplary embodiments of the present invention, with reference to the drawings. Common reference numerals across the drawings indicate same parts unless explicitly stated otherwise. Moreover, each member and each site as represented in the drawings are merely schematic representations thereof, and sizes and positional relationships of an actual product are not necessarily accurately represented therein.

### (1) First Element

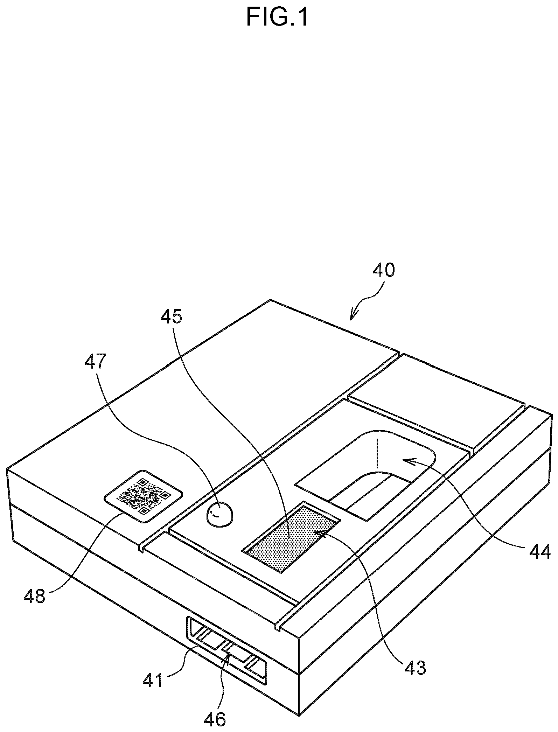

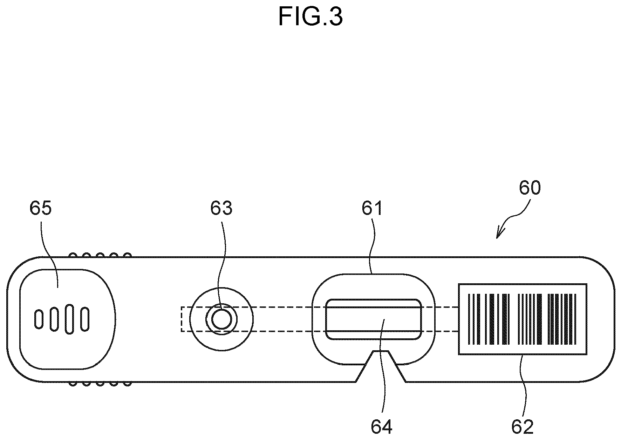

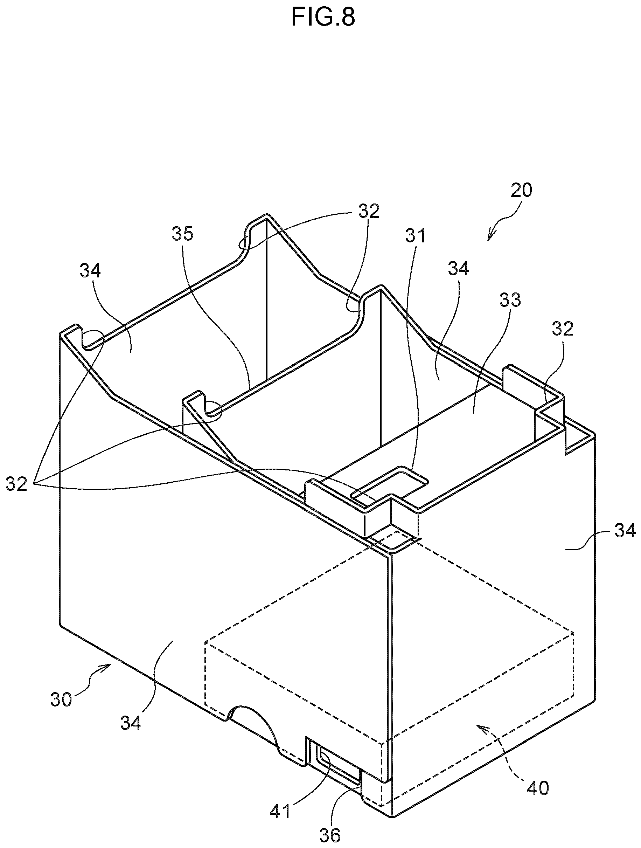

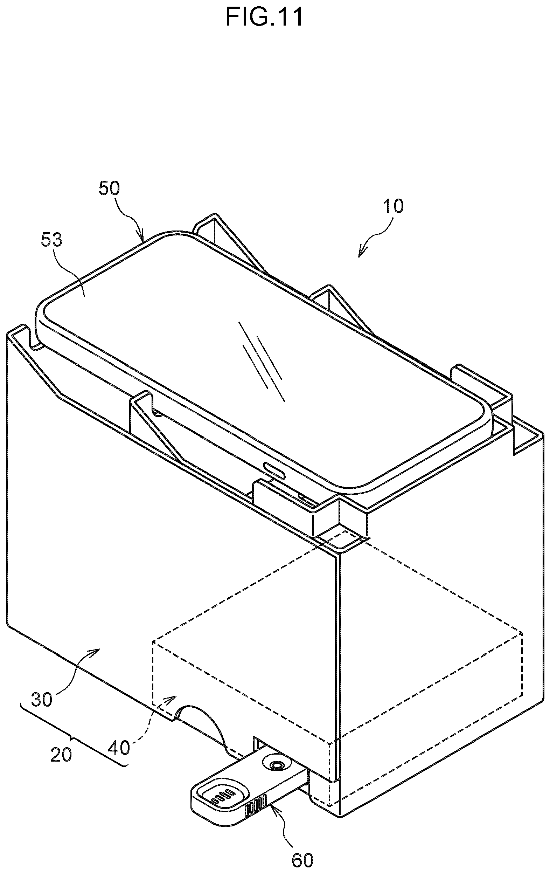

Fig. 1 is an upper perspective view of a holder 40 serving as a first element employed in a measurement system 10 of the present exemplary embodiment (see Fig. 11). The measurement system 10 may include a holder 40 such as illustrated in Fig. 1 as a member configuring (providing) a housing 20 (see Fig. 8). The holder 40 has a box shape and is formed with two openings, i.e., a measurement opening section 43 and an identification opening section 44, in an upper face of the holder 40. A filter 45 with characteristics described later is fitted into the measurement opening section 43. A sensor 47 for sensing light is furthermore provided at the upper face of the holder 40. An insertion port 41 into which a test sample carrier 60 described later (see Fig. 3) is to be inserted opens onto a side face of the holder 40. The interior space of the insertion port 41 is a space in communication with both the measurement opening section 43 and the identification opening section 44, and configures a housing section 46 that is a space in which a part of the test sample carrier 60 is housed. Moreover, identification information 48 of the holder 40 serving as a first element is displayed in the vicinity of the measurement opening section 43 on the upper face of the holder 40 by, for example, a QR code (registered trademark). The holder 40 serving as a first element is configured such that the test sample carrier 60 applied with the measurement target is held therein.

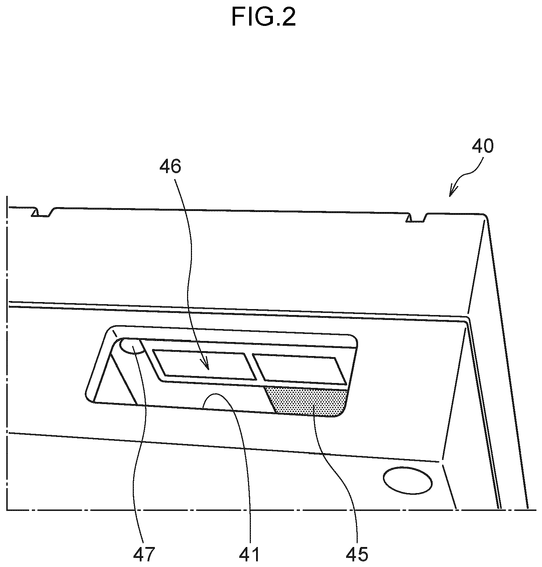

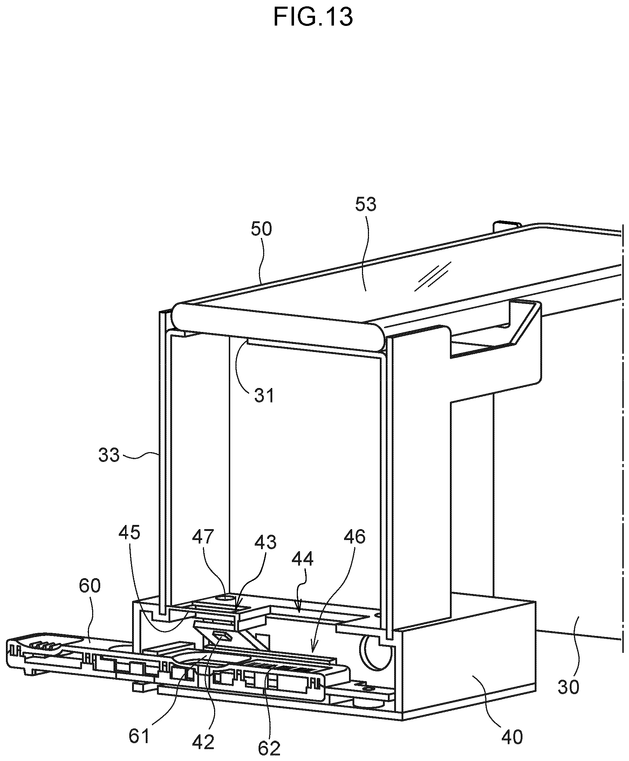

Fig. 2 is a lower perspective view illustrating an enlargement of a vicinity of the insertion port 41 of the holder 40. The sensor 47 is provided in the vicinity of the measurement opening section 43 of the housing section 46 that is the interior space of the insertion port 41. Light rays of a wavelength appropriate for imaging the measurement target are radiated from a light source 42 (see Fig. 13) provided inside the housing section 46. Then, the filter 45 has optical characteristics that suitably transmit only light rays of the wavelength employed for such imaging.

### (2) Test Sample Carrier

Fig. 3 is a plan view of the test sample carrier 60 employed in the measurement system 10 of the present exemplary embodiment (see Fig. 11). The test sample carrier 60 of the present exemplary embodiment is formed as a test strip exhibiting a flat bar shaped external profile. A grip portion 65, an upper face side of which is indented, is formed to one end of the test sample carrier 60. A user can grip the test sample carrier 60 by pinching the grip portion 65 with finger tips. A test paper 64 having a shape elongated along a length direction of the test sample carrier 60 is housed inside the test sample carrier 60. The test paper 64 is exposed toward above at two openings formed in the upper face of the test sample carrier 60. These two openings include a test sample spot application portion 63 near the grip portion 65, and a measurement opening 61 at which measurement of the measurement target is performed further away from the grip portion 65. A test sample carrier identification region 62 recorded with information related to the test sample carrier 60 is formed at the other end side of the test sample carrier 60, namely, on the upper face of a location furthest away from the grip portion 65. Hereafter, a side of the test sample carrier 60 near the grip portion 65 is called an "upstream side", and a side thereof near the test sample carrier identification region 62 is called a "downstream side". Note that the test sample carrier 60 is not limited to a bar shaped test strip such as illustrated in Fig. 3 and, for example, a carrier formed as a plate including a measurement chip or a well may be employed therefor.

The test paper 64 is a water-absorbent body such as filter paper or is configured by (provided by) coating a water-absorbent layer onto a surface of a synthetic resin substrate. A reaction reagent that reacts with the measurement target to develop a color is applied to the test paper 64. A test sample suspected to contain the measurement target is spotted onto the test sample spot application portion 63. Examples of the test sample include a liquid sample collected from a living body such as, for example, blood or urine, or a diluted solution of either that is diluted with an appropriate solvent, or a liquid sample in which a solid matter or mucus collected from a living body either is diluted with an appropriate solvent or the like or suspended therein. Examples of the measurement target include a component contained in a liquid sample, or an antigen or the like derived from a foreign microorganism or virus.

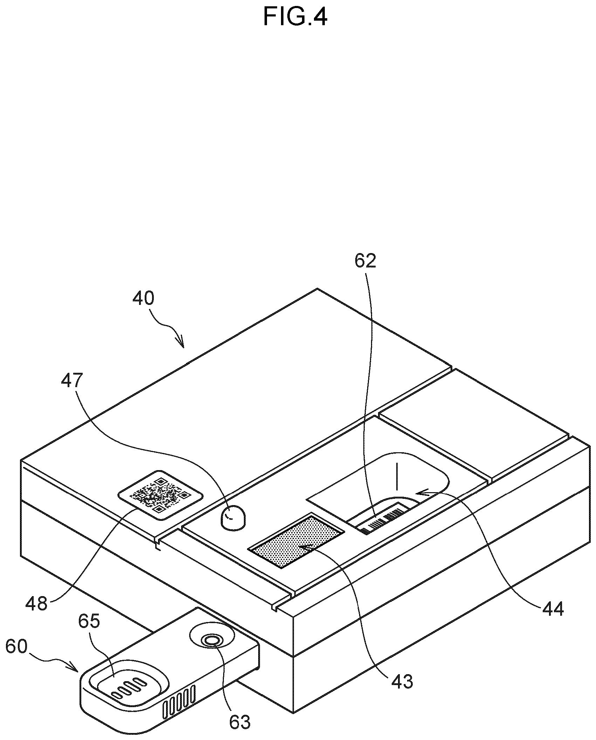

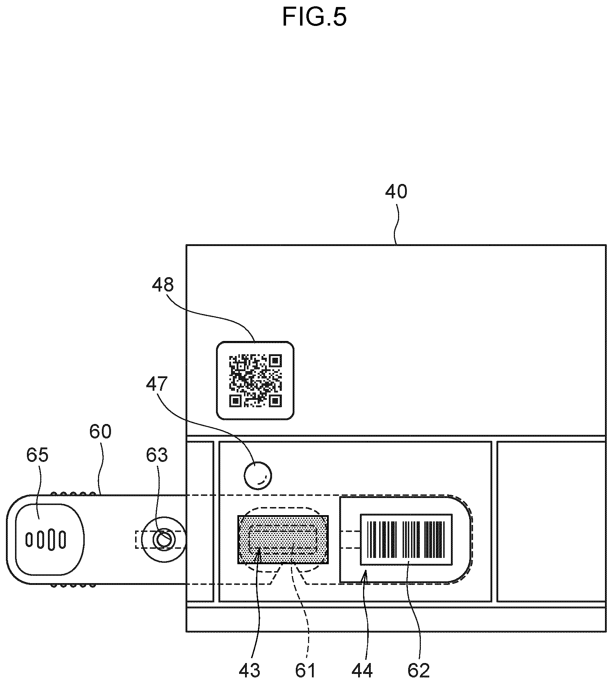

Fig. 4 is an upper perspective view of a state in which the test sample carrier 60 is mounted to the holder 40. Moreover, Fig. 5 is a plan view illustrating this state. As illustrated in Fig. 4 and Fig. 5, the test sample carrier 60 is inserted downstream side first through the insertion port 41 and into the interior of the housing section 46. In this state, as illustrated in Fig. 5, the measurement opening 61 is positioned in the same flat plane as the measurement opening section 43 and, moreover, the test sample carrier identification region 62 is positioned in the same flat plane as the identification opening section 44.

In this state, when a test sample is spotted onto the test sample spot application portion 63, the test sample flows in the downstream direction under capillary action of the test paper 64, such that a control reaction band (not illustrated in the drawings) indicating the spot application of the test sample on the measurement opening 61 occurs near the downstream side. Furthermore, in a case in which the measurement target is contained in the test sample, a target reaction band (not illustrated in the drawings) having a strength according to the concentration of the measurement target occurs near the upstream side relative to the control reaction band. The target reaction band on the test paper 64 is positioned at a central portion of the measurement opening 61, and the control reaction band is positioned at the downstream side outside the central portion. The measurement system 10 of the present exemplary embodiment measures a concentration of the measurement target by radiating light emitted from the light source 42 onto the target reaction band, and measuring the intensity of light generated thereby. The test sample carrier identification region 62 described above is, for example, recorded with (encoded with) which type of test paper 64 is housed in the test sample carrier 60 as identification information that is information related to the test sample carrier 60. Examples of the identification information include a bar code, a QR code (registered trademark), or the like.

### (3) Second Element

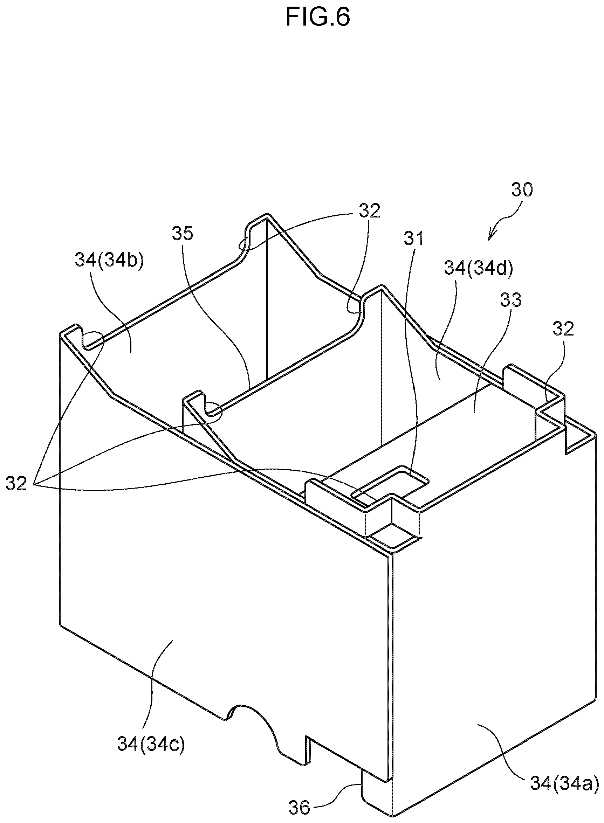

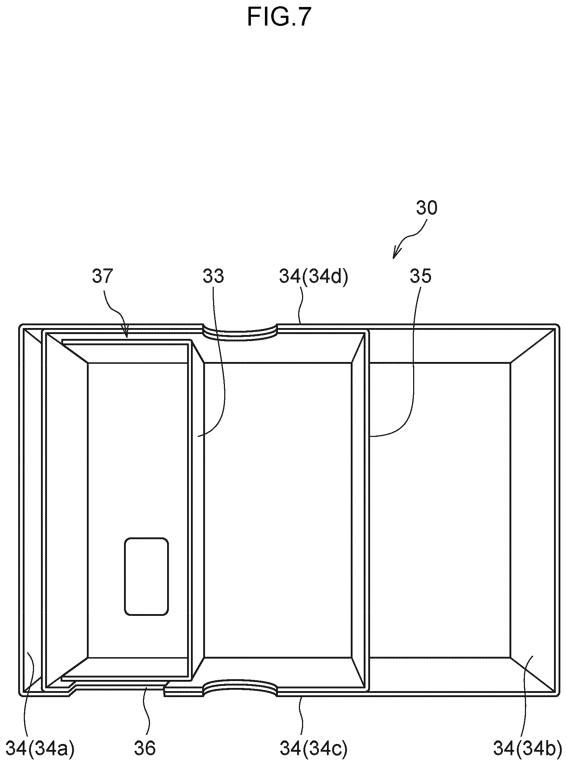

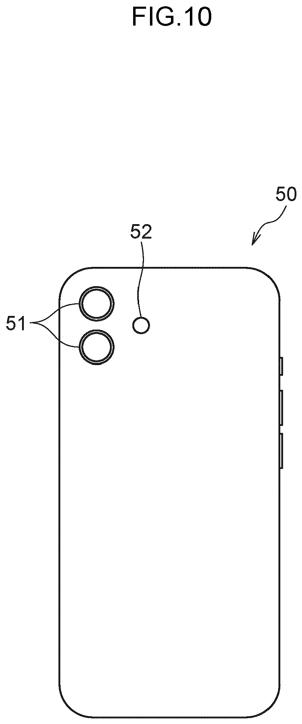

Fig. 6 is an upper perspective view of a casing 30 serving as part of a configuration of the second element employed in the measurement system 10 of the present exemplary embodiment (see Fig. 11). Moreover, Fig. 7 is a bottom view of the casing 30. The casing 30 is configured as a paper box having a substantially cuboidal shape with open upper face and lower face. The casing 30 of the present exemplary embodiment is provided as a product in a flat-folded state, for assembly as a three-dimensional box as illustrated in the drawing when used. The four side faces of the casing 30 configure an external wall section 34 that is vertically upstanding. A placement frame 32 that is a frame for placement of a smart device 50 (see Fig. 10) described later is formed on the upper face of the casing 30. A light blocking section 33 of a box shape having a closed upper face including an open window 31 and an open lower face (see Fig. 7) is formed at one side of the interior of the casing 30 (hereafter referred to as the "front side"). The casing 30 serving as a part of the second element is a configuration to which the holder 40 serving as the first element is assembled.

The four faces of the external wall section 34 include a front face 34a that is a face on which the light blocking section 33 is positioned, a back face 34b that is a face on an opposite side to the front face 34a, a left side face 34c that is a face on a left side when viewed from the front face 34a, and a right side face 34d that is a face on an opposite side to the left side face 34c. The interior of the casing 30 is partitioned by a reinforcement portion 35 that is parallel to the front face 34a and the back face 34b. A rectangular shaped cut-out portion 36 is also formed at a lower edge of the front side of the left side face 34c.

### (4) Housing

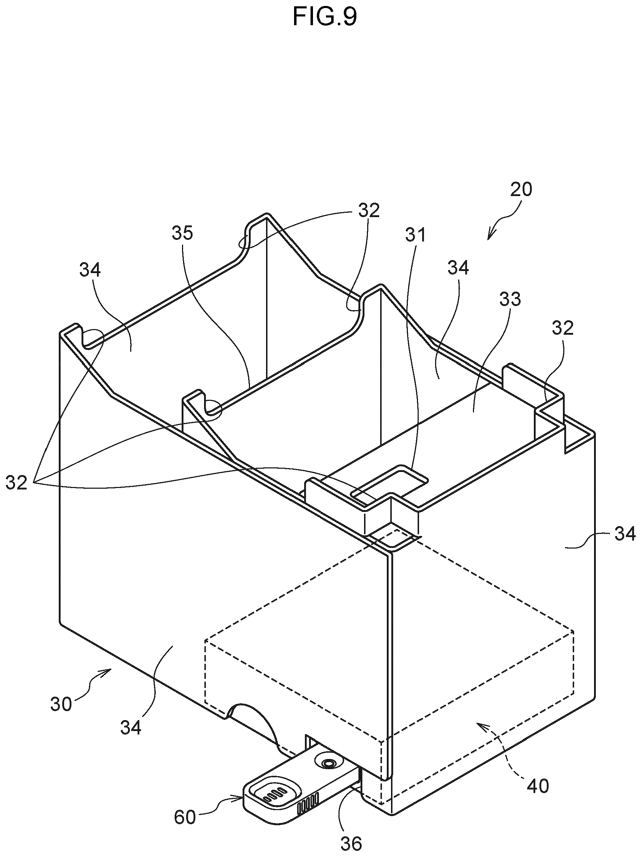

As illustrated in Fig. 7, there is a gap between the lower edge of the light blocking section 33 and the lower edge of the external wall section 34, and a space surrounded on four sides by the front face 34a, the reinforcement portion 35, the left side face 34c, and the right side face 34d, and having a height corresponding to the gap is referred to as a housing area 37. Assembly of the housing 20 illustrated in Fig. 8 is completed by the holder 40 being assembled to the housing area 37 of the casing 30. In this state, the cut-out portion 36 of the casing 30 and the insertion port 41 of the holder 40 are aligned with each other. A state in which the test sample carrier 60 is mounted to the insertion port 41 as illustrated in Fig. 4 and Fig. 5 in this state is as illustrated by the upper perspective view of Fig. 9.

### (5) Smart Device

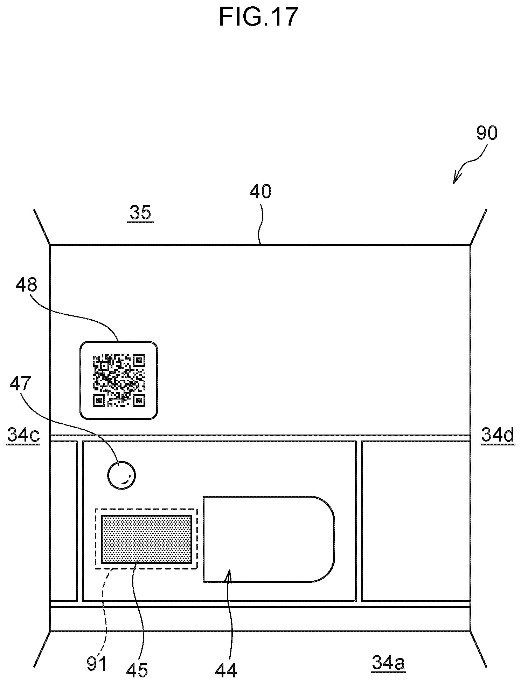

Fig. 10 is a bottom view of the smart device 50 when employed in the measurement system 10 of the present exemplary embodiment (see Fig. 11) in a state placed in the housing 20. Herein, the configuration resulting from the smart device 50 being placed in the casing 30 is the second element. Although, in the present exemplary embodiment, a smartphone is employed as the smart device 50, a tablet terminal with a camera function may be employed as the smart device 50. An imaging section 51 configured by (comprising) a camera, and an illuminator 52 configured by (comprising) a flash that radiates visible light in cooperation therewith, are provided at a bottom face side (so-called back face) of the smart device 50. Note that the top face side (so-called front face) of the smart device 50 is configured by a display 53. The illuminator 52 radiates a verification area 90 (see Fig. 17) of the holder that is the first element when acquiring a verification image described later.

### (6) Measurement System

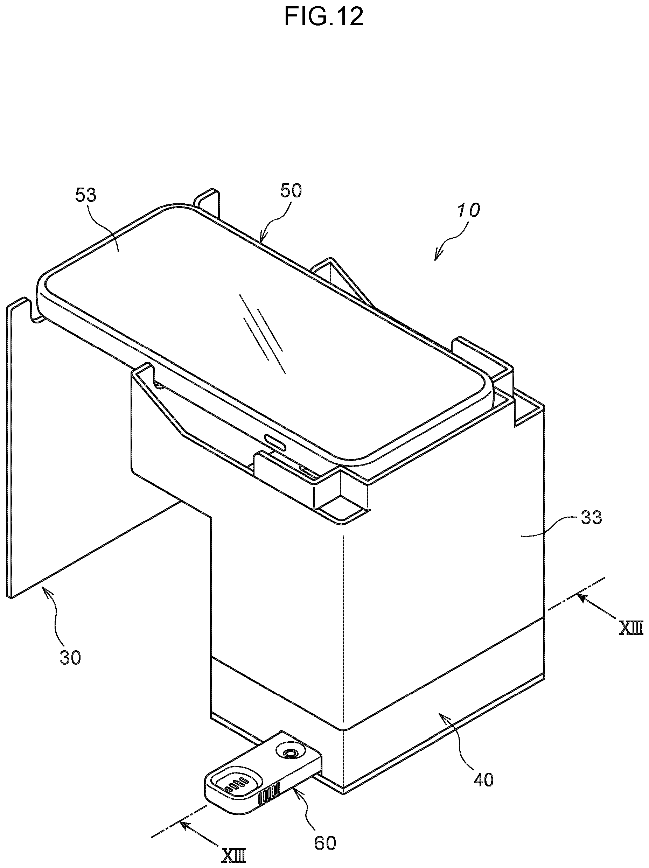

At the inside of the placement frame 32 of the housing 20 illustrated in Fig. 9, the smart device 50 illustrated in Fig. 10 is placed with the display 53 facing upward while the imaging section 51 and the illuminator 52 are aligned with the window 31, and thereby the measurement system 10 of the present exemplary embodiment is configured as illustrated in the upper perspective view of Fig. 11. Entry of external light should be impeded when, from this state, the measurement opening section 43 and the identification opening section 44 of the holder 40 are covered by the light blocking section 33 as illustrated in the upper perspective view of Fig. 12, which illustrates a state in which the front face 34a, the left side face 34c, and the right side face 34d of the external wall section 34 is supposed to be removed. However, sometimes extraneous stray light leaks into the interior of the casing 30 due to a way in which the casing 30 is assembled, or due to a mounting fit between the casing 30 and the holder 40. A description is given later regarding this point.

Moreover, as illustrated in Fig. 13, which illustrates a cross-section taken along line XIII-XIII of Fig. 12, the measurement opening section 43 and the filter 45 are positioned above the measurement opening 61 of the test sample carrier 60, and the identification opening section 44 is positioned above the test sample carrier identification region 62. Furthermore, the light source 42 is installed below and slightly at the back face side of the identification opening section 44 and radiates from diagonally above the measurement opening 61. The window 31 of the casing 30 is positioned directly above the measurement opening section 43, and, as well as the measurement opening section 43, the test sample carrier identification region 62 is also contained in the visual field through this of the imaging section 51 of the smart device 50.

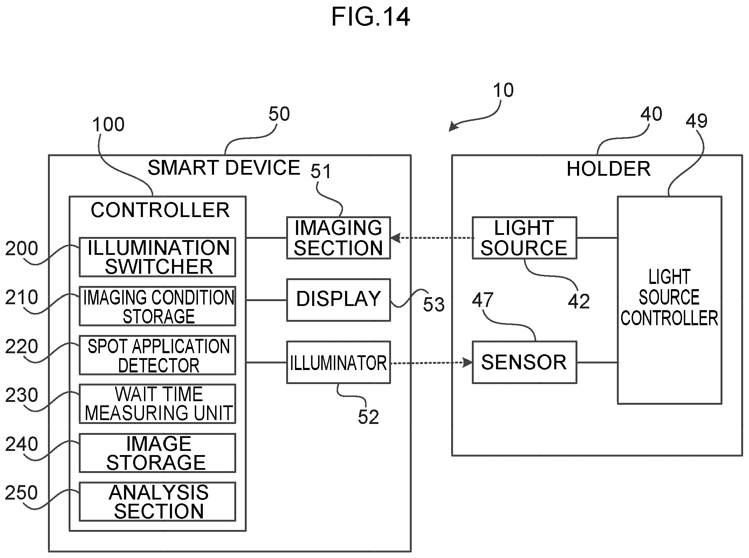

Fig. 14 illustrates the measurement system 10 of the present exemplary embodiment as a functional block diagram. The smart device 50 is provided with the imaging section 51 and the illuminator 52 illustrated in Fig. 10, and the display 53 illustrated in Fig. 11, and also with a controller 100 that controls these. The controller 100 utilizes a CPU 110, ROM 120, RAM 130, and a storage device 150, described later, as hardware resources of a computer to function as each of the following means.

Namely, the controller 100 functions as an illumination switcher 200 that switches illumination by the illumination section 52 ON/OFF (turn-on/turn-off). The illumination switcher 200 may, specifically, be implemented as an application installed on the smart device 50, or alternatively may be implemented as a means that utilizes electrical or optical sensing with the holder 40, or as a wireless communication means (for example, Bluetooth (registered trademark) or the like) with the holder 40. Moreover, the controller 100 functions as an imaging condition storage 210 stored with conditions for capturing with the imaging section 51. Conditions defined as the imaging conditions include, for example, a wait time needed for reaction between the measurement target and the reagent. Moreover, through the imaging section 51 the controller 100 functions as a spot application detector 220 to detect spot application of the test sample on the test sample carrier 60. Moreover, the controller 100 also functions as a wait time measuring unit 230 to measure the wait time. Then, the controller 100 functions as an image storage 240 to store an image of a measurement region 91 (see Fig. 17) containing the measurement opening section 43 as captured by the imaging section 51. Furthermore, the controller 100 functions as an analysis section 250 that captures an image of the image captured by the imaging section 51. The imaging section 51 captures the verification area 90 (see Fig. 17) containing the measurement region 91 in which the measurement target in the test sample applied to the test sample carrier 60 is to be positioned in the holder 40 serving as the first element, and acquires a verification image described later. Moreover, the analysis section 250 analyzes the verification image acquired by the imaging section 51, and detects any error after the holder 40 serving as the first element is assembled to the casing 30 serving as the second element, as described later. In other words, the imaging section 51 and the analysis section 250 are provided at the smart device 50 that is included in the second element. Namely, the second element can be said to be equipped with the imaging section 51.

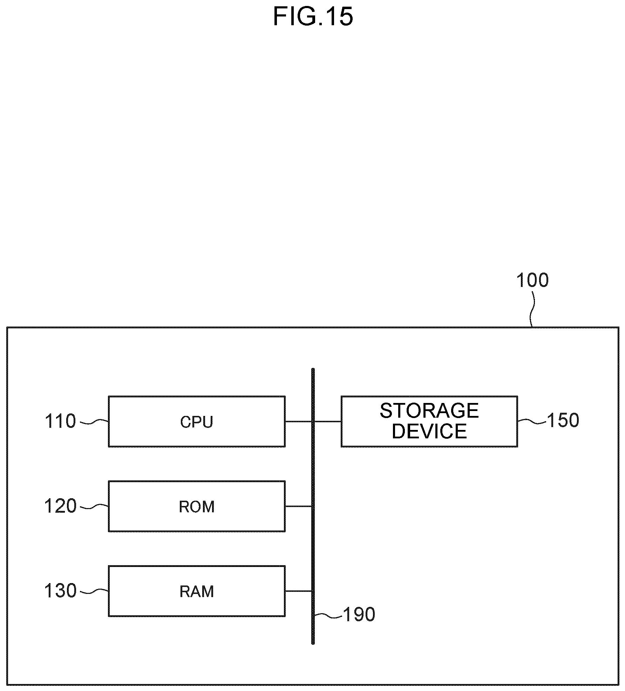

As illustrated by the hardware configuration of Fig. 15, the controller 100 includes the central processing unit (CPU) 110, the read only memory (ROM) 120, the random access memory (RAM) 130, and the storage device 150. These configurations are each connected together through a bus 190 so as to be capable of communicating with each other.

The CPU 110 is a central processing unit that executes various programs and controls each section. Namely, the CPU 110 reads a program from the ROM 120 or the storage device 150, and executes the program using the RAM 130 as a work area. The CPU 110 controls the measurement system 10 according to the program recorded on the ROM 120 or the storage device 150.

The ROM 120 is stored with various programs and various data. The RAM 130 serves as a work area for temporarily storing programs and/or data. The storage device 150 is configured as storage by a hard disk drive (HDD), solid state drive (SSD), flash memory, or the like, and stores various programs including an operating system and various data.

The holder 40 includes the light source 42 that radiates the measurement region 91, the sensor 47 that detects ON/OFF (turn-on/turn-off) of the illuminator 52, and a light source controller 49 that turns the light source 42 on with input with a signal from the sensor 47. The light source controller 49 is configured by hardware resources of a computer similarly to the controller 100 of the smart device 50. Note that as long as the light source controller 49 is able to perform control to turn the light source 42 on when capturing the measurement region 91, described later, then the light source controller 49 may be implemented to turn the light source 42 on irrespective of input mode (for example, wired or wireless) of the signal from the sensor 47. Moreover, the light source controller 49 can control to turn the light source 42 off.

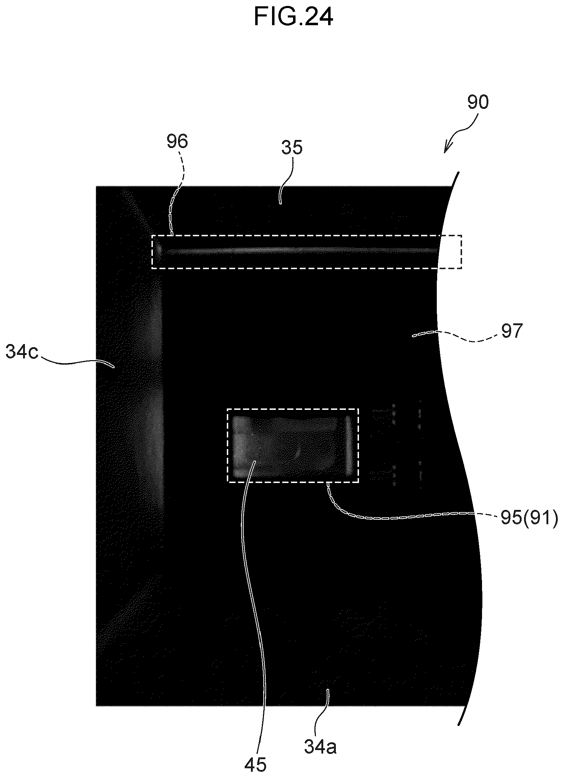

In the measurement system 10 described above, based on the verification image described later that was captured in a state in which the casing 30 serving as the second element configured (provided) a dark chamber, a stray light error is detected by light intensity in, from out of the verification area 90 (see Fig. 17), at least one of a check region 97 (see Fig. 24) in which extraneous stray light is possible to occur or a specific region 96 (see Fig. 24) in which extraneous stray light is likely to occur. In addition, in the measurement system 10 described above, in a state in which the casing 30 serving as the second element configures a dark chamber, preferably a stray light error is detected by comparing the light intensity of the at least one of the check region 97 or the specific region 96 against a predetermined threshold. For example, a stray light error can be determined when an average value of the light intensity of pixels of the check region 97 or the specific region 96 represented by the image is greater than the predetermined threshold. Alternatively, a stray light error can be determined when the number of pixels having light intensity greater than a predetermined threshold is a specific number (for example 10) or greater in the check region 97 or the specific region 96.

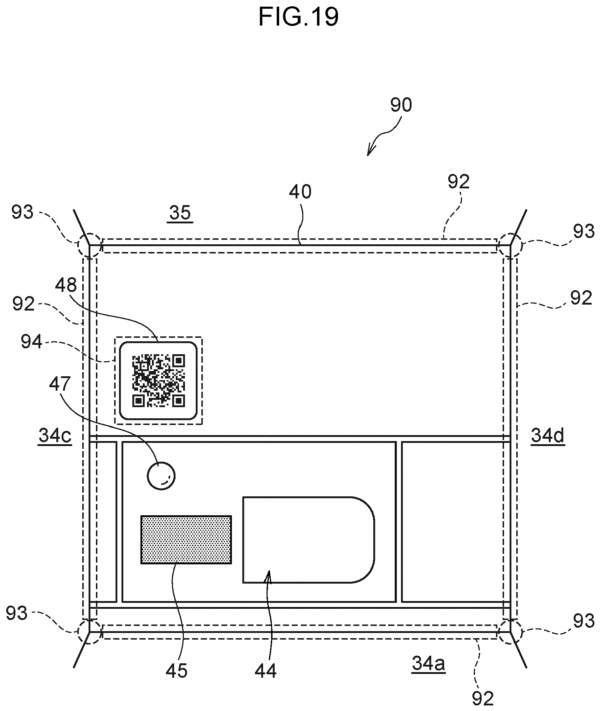

Moreover, in the measurement system 10 described above, the verification area 90 (see Fig. 17) is preferably comprises, in addition to at least the measurement region 91 (see Fig. 17), boundary regions 92 (see Fig. 19) that are boundaries between the holder 40 serving as the first element and the casing 30 serving as the second element, in a configuration in which, from the verification image described later, the analysis section 250 specifies positions on the image in the verification area 90 (see Fig. 19) of at least the boundary regions 92 and detects an assembly error based on the specified positions. In such cases, in the measurement system 10 described above, it is more preferable that there is an identification region 94 (see Fig. 19) displayed with the identification information 48 of the holder 40 serving as the first element inside the verification area 90, and an assembly error is detected based on the position in the image of the identification region 94 in the verification area 90, and, furthermore, it is more preferable to detect whether or not the holder 40 serving as the first element is suitable based on a content of the identification information 48.

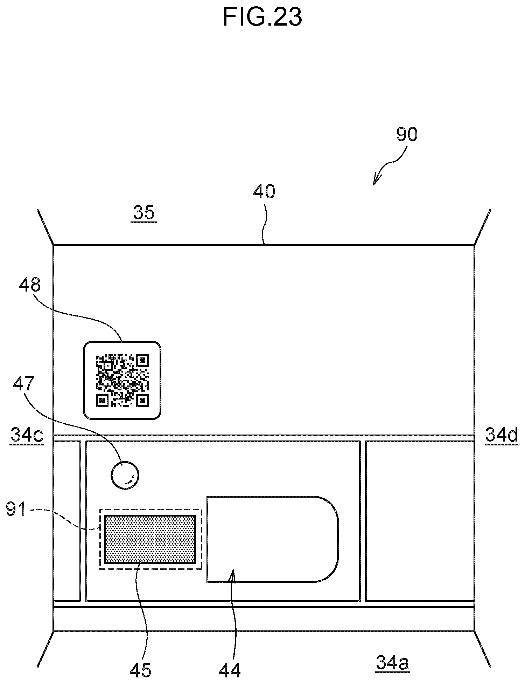

Furthermore, the measurement system 10 described above is preferably configured with the filter 45 partitioning between the test sample carrier 60 and the imaging section 51 provided in the measurement region 91 (see Fig. 23), with errors with the filter 45 detected based on the image information of the filter 45 in the verification image described later.

### (7) Detection of Assembly Error by Measurement System

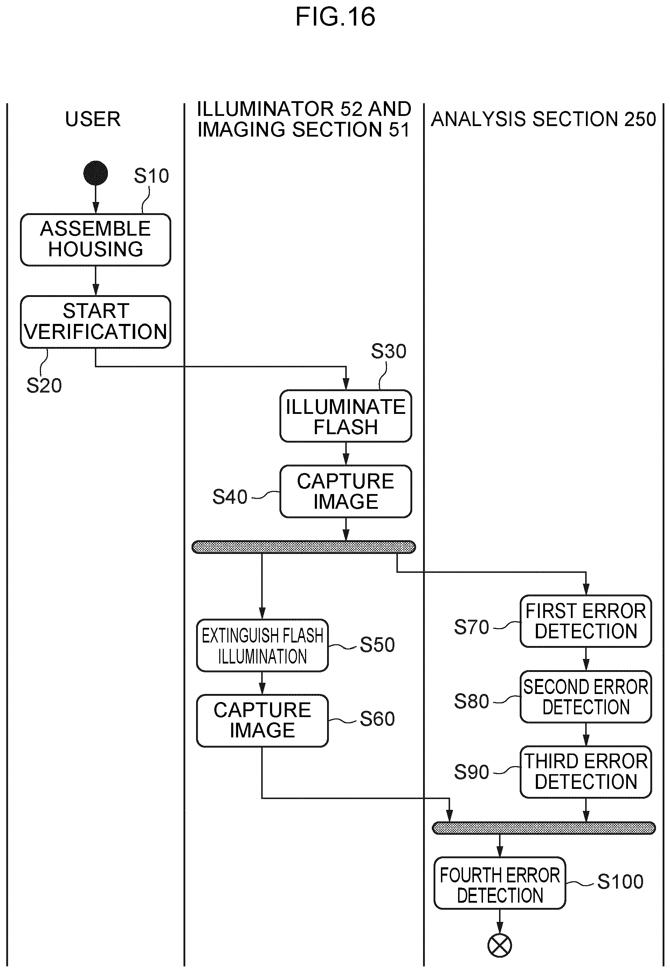

Description follows regarding examples of detection of assembly error using the measurement system 10 of the present exemplary embodiment, with reference to the flowcharts of Fig. 16, Fig. 18, Fig. 20, Fig. 22, and Fig. 25, and with reference to the schematic diagrams of the verification area 90 of Fig. 17, Fig. 19, Fig. 21, Fig. 23, and Fig. 24.

Fig. 16 is a flowchart illustrating an overview of error detection in a measurement system of an exemplary embodiment. In the three vertically partitioned columns in this figure, processes executed by a user are indicted in the left column, processes executed by the illuminator 52 and the imaging section 51 are indicted in the central column, and processes executed by the analysis section 250 are indicated in the right column.

Firstly, in the process indicated by S10, the user assembles the casing 30 serving as the second element into a three-dimensional box as illustrated in Fig. 6. Then, the housing 20 illustrated in Fig. 8 is assembled by assembling the holder 40 serving as the first element to the casing 30. Then, in a state in which the test sample carrier 60 is not mounted to the holder 40, the smart device 50 illustrated in Fig. 10 is placed at the position in the housing 20 illustrated in Fig. 11. In the process indicated by S20, by the user operating the smart device 50 in this state, illumination of the interior of the casing 30 is performed by the illuminator 52, and imaging is performed by the imaging section 51.

Namely, in the process indicated by S30, the illumination switcher 200 of the controller 100 (see Fig. 14) causes a flash of the illuminator 52 to turn on. At the same time as the flash illumination, in the process indicated by S40 the imaging section 51 images an image, as illustrated in Fig. 17, of the verification area 90 containing the upper face of the holder 40 and the front face 34a, the left side face 34c, the right side face 34d, and the lower edge portion of the reinforcement portion 35 that surround the upper face of the holder 40 on four sides. This image includes the measurement region 91 that is an area in which measurement of the measurement target is performed when the test sample carrier 60 is inserted. This image is temporarily stored as a verification image in the RAM 130 or the storage device 150 of the controller 100.

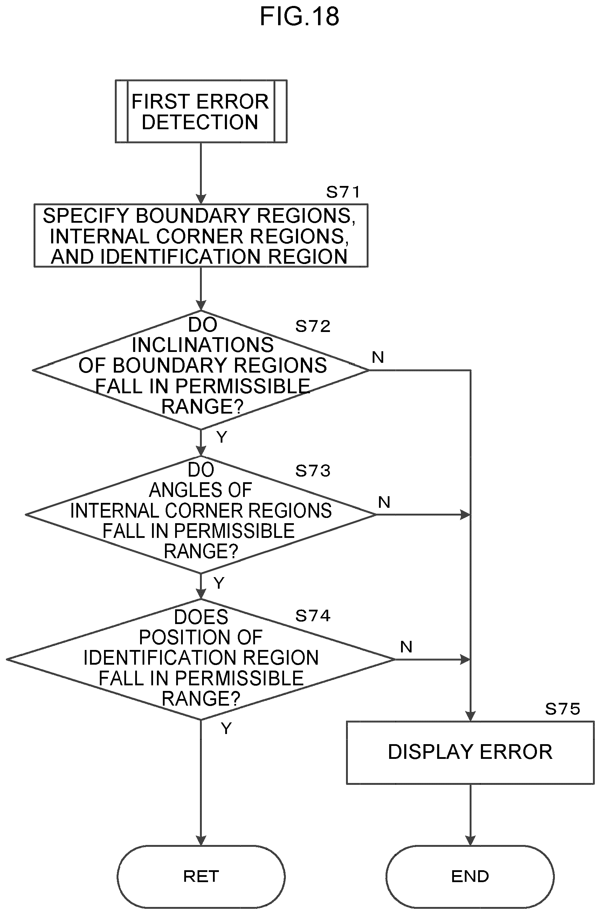

Next, the analysis section 250 executes a first error detection process indicated by S70 based on the verification image of the verification area 90 illustrated in Fig. 17. The first error detection process is a process to detect an error in the assembly state, and an overview thereof is illustrated in the flowchart illustrated in Fig. 18.

First, at a stage indicated by S71, the boundary regions 92, internal corner regions 93, and the identification region 94 as illustrated in Fig. 19 are specified in the verification area 90 by the analysis section 250. Herein, the boundary region 92 is specified as a region corresponding to a boundary between the holder 40 serving as the first element and the casing 30 serving as the second element, that is, a region corresponding to the four sides of the holder 40. Moreover, the internal corner regions 93 are specified as regions corresponding to the four corners between adjacent boundary regions of the boundary regions 92. The identification region 94 is specified as a region surrounding the identification information 48.

Next, at the stage indicated by S72, the analysis section 250 verifies whether or not an inclination of each of the boundary regions 92 falls within a permissible range. Ideally, the boundary regions 92 are configured by pairs of two boundary regions 92 respectively exhibiting a substantially straight line shape that face each other and should be parallel to each other, but they may be configured otherwise depending on how the housing 20 is assembled. Thus, a numerical range expressing a permissible amount of deviation from such an ideal state is stored as a permissible range in the storage device 150 of the controller 100. Processing transitions to the stage indicated by S75 in cases in which the inclination of the boundary regions 92 is determined not to fall within the permissible range, and an error message stating that assembly is not correct is displayed on the display 53 of the smart device 50 by the controller 100.

In cases in which the inclinations of the boundary regions 92 are determined to fall within the permissible range at the stage indicated by S72, verification is performed by the analysis section 250 at the stage indicated by S73 as to whether or not angles of the internal corner regions 93 fall within a permissible range. The internal corner regions 93 each ideally exhibit a right angle, but may be otherwise due to how the housing 20 is assembled. Thus, a numerical range expressing a permitted amount of deviation from such an ideal state is stored as a permissible range in the storage device 150 of the controller 100. Processing proceeds to the stage indicated by S75 in cases in which the angles of the internal corner regions 93 are determined not to fall within the permissible range, and an error message stating that assembly is not correct is displayed on the display 53 of the smart device 50 by the controller 100.

In cases in which the angles of the internal corner regions 93 are determined to fall within the permissible range at the stage indicated by S73, whether or not the position of the identification region 94 falls within the permissible range is verified by the analysis section 250 at the stage indicated by S74. A numerical range expressing a permissible amount of deviation permitted from the position the identification region 94 should be at in the verification area 90 is stored as a permissible range in the storage device 150 of the controller 100. Processing proceeds to a second error detection process (S80) of Fig. 16 in cases in which the position of the identification region 94 was determined to fall within the permissible range. On the other hand, processing proceeds to a stage indicated by S75 in cases in which the position of the identification region 94 was determined not to fall within the permissible range, and an error message stating that assembly is not correct is displayed on the display 53 of the smart device 50 by the controller 100.

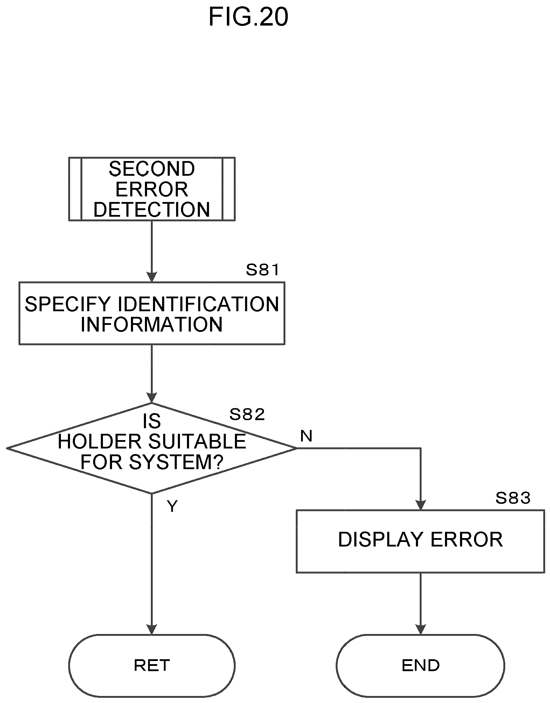

In cases in which the position of the identification region 94 was determined to fall within the permissible range at the stage indicated by S74, the analysis section 250 executes the second error detection process indicated by S80 of Fig. 16 based on the verification image of the verification area 90 illustrated in Fig. 17. The second error detection process is a process to detect whether or not the holder 40 serving as the first element is suitable, and an overview thereof is illustrated in the flowchart illustrated in Fig. 20.

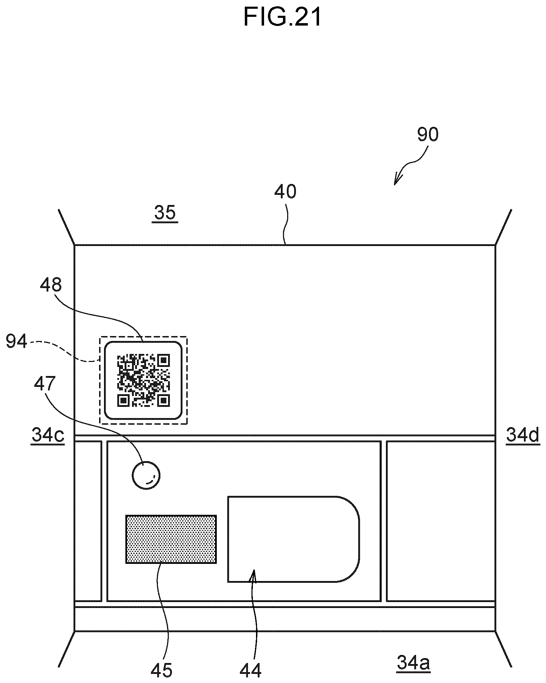

First, at the stage indicated by S81, the identification information 48 illustrated in Fig. 21 is specified inside the verification area 90 by the analysis section 250. Specifically, the analysis section 250 specifies information related to the holder 40 serving as the mounted first element (for example, product number, application, usage limitation, or the like) in the identification information 48 present in the identification region 94.

Next, at the stage indicated by S82, whether or not the holder 40 serving as the mounted first element is suitable for the measurement system 10 is determined by the analysis section 250. Processing proceeds to a third error detection process (S90) of Fig. 16 when determined suitable. On the other hand, processing proceeds to the stage indicated by S83 when determined unsuitable, and an error message that the holder 40 is unsuitable is displayed on the display 53 of the smart device 50 by the controller 100.

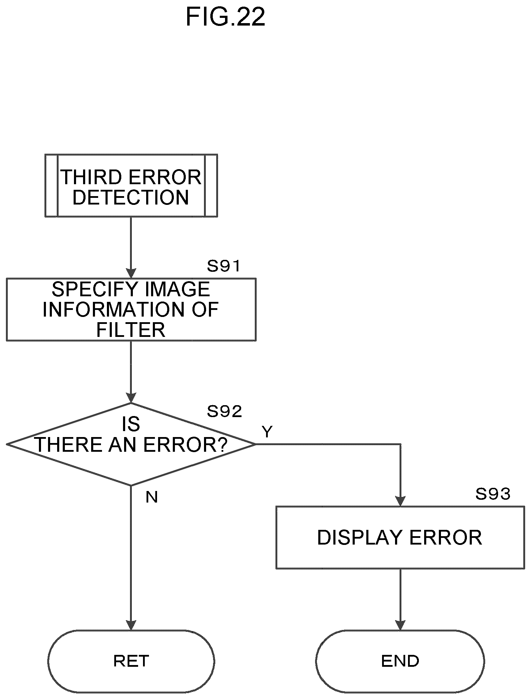

In cases in which the holder 40 serving as the first element is determined suitable for the measurement system 10 at the stage indicated by S82, the analysis section 250 executes the third error detection process indicated by S90 of Fig. 16 based on the verification image of the verification area 90 illustrated in Fig. 17. The third error detection process is a process to detect a filter error 45, and an overview thereof is illustrated in the flowchart illustrated in Fig. 22.

First, at the stage indicated by S91, image information of the filter 45 is specified by the analysis section 250 based on the image of the measurement region 91 illustrated in Fig. 23 in the verification area 90.

Next, at the stage indicated by S92, whether or not there is an error is determined by the analysis section 250 from the image information of the filter 45. Assumed examples of such an error include scratches, adhered finger print, or the like. For example, such an error is determined to be present by the analysis section 250 when there is non-uniform image data in the image information of the filter 45. Processing proceeds to a fourth error detection process of Fig. 16 (S100) in cases in which there is no error with the filter 45. On the other hand, processing proceeds to the stage indicated by S93 in cases in which there is any error with the filter 45, and an error message that there is an error with the filter 45 is displayed on the display 53 of the smart device 50 by the controller 100.

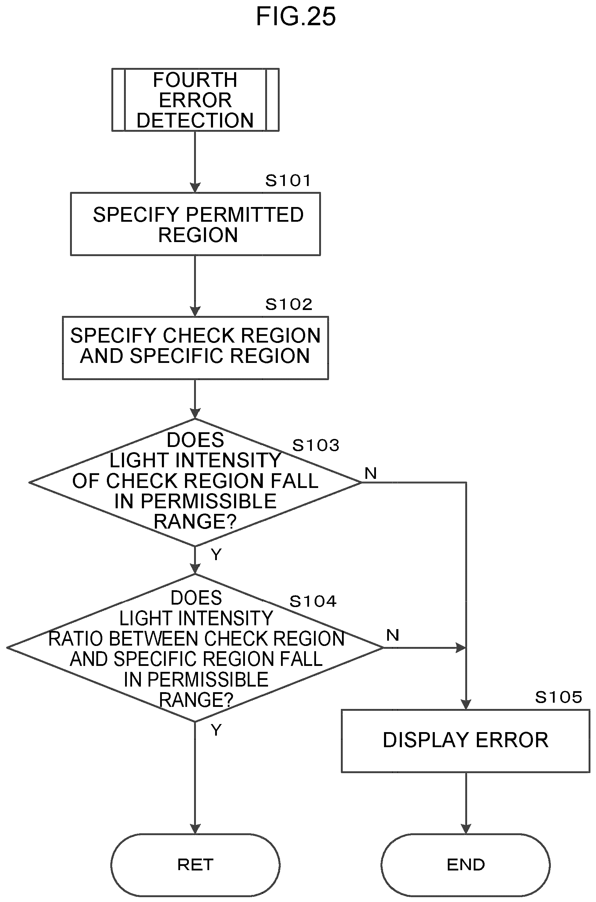

After an image is captured in the process indicated by S40 of Fig. 16, in the process indicated by S50 the illumination switcher 200 of the controller 100 (see Fig. 14) turns off the flash of the illuminator 52. After turning the flash off, in the process indicated by S60 the imaging section captures an image of the verification area 90 in a state in which the inside of the casing 30 is a dark chamber as illustrated in Fig. 24. This image includes a permitted region 95 corresponding to the measurement region 91, a specific region 96 corresponding to the boundary between the holder 40 and the reinforcement portion 35, and a check region 97 that is an area of the verification area 90 other than the permitted region 95 and the specific region 96. This image is temporarily stored as a verification image in the RAM 130 or the storage device 150 of the controller 100. The analysis section 250 executes the fourth error detection process of S100 based on the verification image. The fourth abnormality detection process is a process to detect a stray light error, and an overview thereof is illustrated in the flowchart illustrated in Fig. 25.

First, at the stage indicated by S101 the permitted region 95 illustrated in Fig. 24 is specified inside the verification area 90 by the analysis section 250. The permitted region 95 is a region corresponding to the measurement region 91. Herein, the test sample carrier 60 is not mounted to the holder 40 in a state in which the fourth error detection process is being executed, and so external light sometimes enters the housing section 46 (see Fig. 1) through the insertion port 41, and this light is observed in the permitted region 95 as illustrated in Fig. 24. In the fourth error detection process of the present exemplary embodiment the light observed in the permitted region 95 is not treated as being a target for error detection.

Next, at the stage indicated by S102, the specific region 96 illustrated in Fig. 24 is specified in the verification area 90 by the analysis section 250. The specific region 96 is a region in which, for example, a gap between the holder 40 serving as the first element and the casing 30 serving as the second element readily occurs as in the boundary region 92 illustrated in Fig. 19, and is a region in which extraneous stray light readily occurs. Then, the analysis section 250 specifies the check region 97 that is an area of the verification area 90 illustrated in Fig. 24 other than the permitted region 95 and the specific region 96.

Next, at the stage indicated by S103, whether or not the light intensity of the check region 97 falls within the permissible range is verified by the analysis section 250. Specifically, a brightness of the data of each pixel configuring (in the image of) the check region 97 is checked, whether or not the pixel data has a brightness of a specific value or greater is determined, and also deviation from the permissible range can be determined if such pixel data extends across a fixed number or greater of adjacent pixels. Processing proceeds to the stage indicated by S 105 in cases in which the light intensity of the check region 97 is determined not to fall within the permissible range, and an error message that there is extraneous stray light is displayed on the display 53 of the smart device 50 by the controller 100.

Then, at the stage indicated by S104, whether or not a light intensity ratio between the check region 97 and the specific region 96 falls within a permissible range is checked by the analysis section 250. Specifically, the brightness of the data of each pixel configuring (in the image of) the specific region 96 is checked. Next, deviation from the permissible range can be determined in cases in which there are specific number or greater of the specific region 96 pixel data having a brightness of a specific multiple or greater with respect to an average value of the brightness of each pixel data configuring the check region 97. Processing proceeds to the stage indicated by S 105 in cases in which the light intensity ratio between the check region 97 and the specific region 96 is determined to not fall within the permissible range, and an error message that there is extraneous stray light is displayed on the display 53 of the smart device 50 by the controller 100.

After any error is not detected by the analysis section 250 from the first error detection process to the fourth error detection process, the test sample carrier 60 is mounted to the housing 20, and measurement of the measurement target in the sample is executed by the measurement system 10, but detailed explanation thereof is omitted herein.

### Industrial Applicability

The present invention is applicable to a measurement system that optically measures a measurement target.

## Claims

1. A measurement system (10) comprising:
a first element in which a test sample carrier (60), applied with a test sample suspected to contain a measurement target, is to be held;
a second element to which the first element is assembled;
an imaging section (51) configured to capture a verification area (90) containing a measurement region (91) in which the measurement target in the test sample applied to the test sample carrier (60) is to be positioned in the first element, and configured to acquire a verification image; and
an analysis section (250) configured to analyze the verification image and detect an assembly error of the first element, **characterized in that**:
the analysis section is configured to detect a stray light error by light intensity in at least one of a check region (97) in the verification area (90) or a specific region (96) in the verification area (90), an extraneous stray light being possible to occur in the check region (97) and being likely to occur in the specific region (96), based on the verification image captured in a state in which the second element is a dark chamber.

2. The measurement system (10) of claim 1, wherein the stray light error is detected by comparing the light intensity in at least one of the check region (97) or the specific region (96) to a predetermined threshold value in a state in which the second element is a dark chamber.

3. The measurement system (10) of claim 1 or 2, wherein:
the verification area (90) includes, in addition to the measurement region (91), at least a boundary region (92) that is a boundary between the first element and the second element; and
the analysis section (250) is configured to specify a position of at least the boundary region (92) in the verification area (90) from the verification image, and is configured to detect the assembly error based on the specified position.

4. The measurement system (10) of any of claim 1 to claim 3, wherein:
an identification region (94) in which identification information (48) of the first element is indicated is provided in the verification area (90); and
the analysis section (250) is configured to detect the assembly error based on a position in an image of the identification region (94) in the verification area (90).

5. The measurement system (10) of any of claim 1 to claim 4, wherein:
a filter (45) separating the test sample carrier (60) from the imaging section (51) is provided in the measurement region (91); and
the analysis section (250) is configured to detect a filter error based on image information of the filter (45) in the verification image.

6. The measurement system (10) of claim 1, wherein:
the verification area (90) includes, in addition to the measurement region (91), at least a boundary region (92) that is a boundary between the first element and the second element; and
the analysis section (250) is configured to, prior to detecting the stray light error, specify a position in an image of at least the boundary region (92) in the verification area (90) from the verification image captured in a state in which the second element is being illuminated by an illuminator, and is configured to detect the assembly error based on the specified position.

7. The measurement system (10) of claim 1 or claim 6, wherein:
an identification region (94) in which identification information (48) of the first element is indicated is provided in the verification area (90); and
the analysis section (250) is configured to, prior to detecting the stray light error, detect the assembly error based on a position in an image of the verification area (90) in the identification region (94) captured in a state in which the second element is being illuminated by an illuminator.

8. The measurement system (10) of claim 4 or claim 7, wherein whether or not the first element is suitable is detected based on a content of the identification information (48).

9. The measurement system (10) of claim 1, claim 2, claim 6 or claim 7, wherein:
a filter (45) separating the test sample carrier (60) from the imaging section (51) is provided in the measurement region (91); and
the analysis section (250) is configured to, prior to detecting the stray light error, detect a filter error based on image information of the filter (45) in the verification image captured in a state in which the second element is being illuminated by an illuminator (52).

10. The measurement system (10) of any of claim 1 to claim 9, wherein the second element includes the imaging section (51).

11. The measurement system (10) of any of claim 1 to claim 9, wherein the imaging section (51) and the analysis section (250) are provided at a smart device (50) that is included in the second element.

12. The measurement system (10) of any of claim 1 to claim 9, wherein the second element includes a casing (30) and a smart device (50).

## Patentansprüche

1. Messsystem (10), umfassend:
ein erstes Element, in dem ein Testprobenträger (60), auf den eine Testprobe aufgebracht ist, bei der vermutet wird, dass sie einen Zielanalyt enthält, zu halten ist;
ein zweites Element, an dem das erste Element montiert ist;
einen Bildaufnahmeabschnitt (51), der dazu konfiguriert ist, einen Verifikationsbereich (90), der einen Messbereich (91) enthält, in dem der Zielanalyt in der auf den Testprobenträger (60) aufgebrachten Testprobe in dem ersten Element zu positionieren ist, zu erfassen, und dazu konfiguriert ist, ein Verifikationsbild zu erfassen; und
einen Analyseabschnitt (250), der dazu konfiguriert ist, das Verifikationsbild zu analysieren und einen Montagefehler des ersten Elements zu detektieren, **dadurch gekennzeichnet, dass**:
der Analyseabschnitt dazu konfiguriert ist, einen Streulichtfehler anhand der Lichtintensität in mindestens einem von einem Prüfbereich (97) in dem Verifikationsbereich (90) oder einem spezifischen Bereich (96) in dem Verifikationsbereich (90) zu detektieren, wobei in dem Prüfbereich (97) ein Auftreten von äußerem Streulicht möglich ist und das Auftreten dessen in dem spezifischen Bereich (96) wahrscheinlich ist, auf der Grundlage des Verifikationsbildes, das in einem Zustand aufgenommen ist, in dem das zweite Element eine Dunkelkammer ist.

2. Messsystem (10) nach Anspruch 1, wobei der Streulichtfehler detektiert wird, indem die Lichtintensität in mindestens einem von dem Prüfbereich (97) oder dem spezifischen Bereich (96) mit einem vorbestimmten Schwellwert in einem Zustand verglichen wird, in dem das zweite Element eine Dunkelkammer ist.

3. Messsystem (10) nach Anspruch 1 oder 2, wobei:
der Verifikationsbereich (90), zusätzlich zu dem Messbereich (91), mindestens einen Grenzbereich (92) einschließt, der eine Grenze zwischen dem ersten Element und dem zweiten Element ist; und
der Analyseabschnitt (250) dazu konfiguriert ist, aus dem Verifikationsbild eine Position von mindestens dem Grenzbereich (92) in dem Verifikationsbereich (90) zu spezifizieren, und dazu konfiguriert ist, den Montagefehler auf der Grundlage der spezifizierten Position zu detektieren.

4. Messsystem (10) nach einem von Anspruch 1 bis Anspruch 3, wobei:
ein Identifikationsbereich (94), in dem Identifikationsinformation (48) des ersten Elements angegeben ist, in dem Verifikationsbereich (90) vorgesehen ist; und
der Analyseabschnitt (250) dazu konfiguriert ist, den Montagefehler auf der Grundlage der Position in einem Bild des Identifikationsbereichs (94) im Verifikationsbereich (90) zu detektieren.

5. Messsystem (10) nach einem von Anspruch 1 bis Anspruch 4, wobei:
ein Filter (45), der den Testprobenträger (60) von dem Bildaufnahmeabschnitt (51) trennt, in dem Messbereich (91) vorgesehen ist; und
der Analyseabschnitt (250) dazu konfiguriert ist, einen Filterfehler auf der Grundlage von Bildinformation des Filters (45) in dem Verifikationsbild zu detektieren.

6. Messsystem (10) nach Anspruch 1, wobei:
der Verifikationsbereich (90), zusätzlich zu dem Messbereich (91), mindestens einen Grenzbereich (92) einschließt, der eine Grenze zwischen dem ersten Element und dem zweiten Element ist; und
der Analyseabschnitt (250) dazu konfiguriert ist, vor dem Detektieren des Streulichtfehlers, aus dem Verifikationsbild, das in einem Zustand aufgenommen ist, in dem das zweite Element durch einen Beleuchter beleuchtet wird, die Position in einem Bild von zumindest dem Grenzbereich (92) im Verifikationsbereich (90) zu spezifizieren, und dazu konfiguriert ist, den Montagefehler auf der Grundlage der spezifizierten Position zu detektieren.

7. Messsystem (10) nach Anspruch 1 oder Anspruch 6, wobei:
ein Identifikationsbereich (94), in dem Identifikationsinformation (48) des ersten Elements angegeben ist, in dem Verifikationsbereich (90) vorgesehen ist; und
der Analyseabschnitt (250) dazu konfiguriert ist, vor dem Detektieren des Streulichtfehlers den Montagefehler auf der Grundlage der Position in einem Bild des Verifikationsbereichs (90) im Identifikationsbereich (94) zu detektieren, das in einem Zustand aufgenommen ist, in dem das zweite Element durch einen Beleuchter beleuchtet wird.

8. Messsystem (10) nach Anspruch 4 oder Anspruch 7, wobei auf der Grundlage von einem Inhalt der Identifikationsinformation (48) detektiert wird, ob das erste Element geeignet ist oder nicht.

9. Messsystem (10) nach Anspruch 1, Anspruch 2, Anspruch 6 oder Anspruch 7, wobei:
ein Filter (45), der den Testprobenträger (60) von dem Bildaufnahmeabschnitt (51) trennt, in dem Messbereich (91) vorgesehen ist; und
der Analyseabschnitt (250) dazu konfiguriert ist, vor dem Detektieren des Streulichtfehlers einen Filterfehler auf der Grundlage von Bildinformation des Filters (45) in dem Verifikationsbild zu detektieren, das in einem Zustand aufgenommen ist, in dem das zweite Element durch einen Beleuchter (52) beleuchtet wird.

10. Messsystem (10) nach einem von Anspruch 1 bis Anspruch 9, wobei das zweite Element den Bildaufnahmeabschnitt (51) einschließt.

11. Messsystem (10) nach einem von Anspruch 1 bis Anspruch 9, wobei der Bildaufnahmeabschnitt (51) und der Analyseabschnitt (250) an einer smarten Vorrichtung (50) vorgesehen sind, die in dem zweiten Element eingeschlossen ist.

12. Messsystem (10) nach einem von Anspruch 1 bis Anspruch 9, wobei das zweite Element ein Gehäuse (30) und eine smarte Vorrichtung (50) einschließt.

## Revendications

1. Système de mesure (10) comprenant :
un premier élément dans lequel un support d'échantillon d'essai (60), sur lequel est appliqué un échantillon d'essai suspecté de contenir une cible de mesure, doit être maintenu ;
un deuxième élément auquel le premier élément est assemblé ;
une section d'imagerie (51) configurée pour capturer une zone de vérification (90) contenant une région de mesure (91) dans laquelle la cible de mesure dans l'échantillon d'essai appliqué au support d'échantillon d'essai (60) doit être positionnée dans le premier élément, et configurée pour acquérir une image de vérification ; et
une section d'analyse (250) configurée pour analyser l'image de vérification et
détecter une erreur d'assemblage du premier élément, **caractérisé en ce que** :
la section d'analyse est configurée pour détecter une erreur de lumière parasite par l'intensité lumineuse dans au moins une région parmi une région de contrôle (97) dans la zone de vérification (90) ou une région spécifique (96) dans la zone de vérification (90), une lumière parasite extérieure pouvant se produire dans la région de contrôle (97) et étant susceptible de se produire dans la région spécifique (96), sur la base de l'image de vérification capturée dans un état dans lequel le deuxième élément est une chambre noire.

2. Système de mesure (10) selon la revendication 1, dans lequel l'erreur de lumière parasite est détectée par comparaison de l'intensité lumineuse dans au moins une région parmi la région de contrôle (97) ou la région spécifique (96) à une valeur de seuil prédéterminée dans un état dans lequel le deuxième élément est une chambre noire.

3. Système de mesure (10) selon la revendication 1 ou la revendication 2, dans lequel :
la zone de vérification (90) inclut, en plus de la région de mesure (91), au moins une région limite (92) qui est une limite entre le premier élément et le deuxième élément ; et
la section d'analyse (250) est configurée pour spécifier une position d'au moins la région limite (92) dans la zone de vérification (90) à partir de l'image de vérification, et est configurée pour détecter l'erreur d'assemblage sur la base de la position spécifiée.

4. Système de mesure (10) selon l'une quelconque des revendications 1 à 3, dans lequel :
une région d'identification (94) dans laquelle une information d'identification (48) du premier élément est indiquée est prévue dans la zone de vérification (90) ; et
la section d'analyse (250) est configurée pour détecter l'erreur d'assemblage sur la base d'une position dans une image de la région d'identification (94) dans la zone de vérification (90).

5. Système de mesure (10) selon l'une quelconque des revendications 1 à 4, dans lequel :
un filtre (45) séparant le support d'échantillon d'essai (60) de la section d'imagerie (51) est prévu dans la région de mesure (91) ; et
la section d'analyse (250) est configurée pour détecter une erreur de filtre sur la base d'informations d'image du filtre (45) dans l'image de vérification.

6. Système de mesure (10) selon la revendication 1, dans lequel :
la zone de vérification (90) inclut, en plus de la région de mesure (91), au moins une région limite (92) qui est une limite entre le premier élément et le deuxième élément ; et
la section d'analyse (250) est configurée pour, avant de détecter l'erreur de lumière parasite, spécifier une position dans une image d'au moins la région limite (92) dans la zone de vérification (90) à partir de l'image de vérification capturée dans un état dans lequel le deuxième élément est éclairé par un illuminateur, et est configurée pour détecter l'erreur d'assemblage sur la base de la position spécifiée.

7. Système de mesure (10) selon la revendication 1 ou la revendication 6, dans lequel :
une région d'identification (94) dans laquelle une information d'identification (48) du premier élément est indiquée est prévue dans la zone de vérification (90) ; et
la section d'analyse (250) est configurée pour, avant de détecter l'erreur de lumière parasite, détecter l'erreur d'assemblage sur la base d'une position dans une image de la zone de vérification (90) dans la région d'identification (94) capturée dans un état dans lequel le deuxième élément est éclairé par un illuminateur.

8. Système de mesure (10) selon la revendication 4 ou la revendication 7, dans lequel le fait de savoir si le premier élément est approprié ou non est détecté sur la base d'un contenu de l'information d'identification (48).

9. Système de mesure (10) selon la revendication 1, la revendication 2, la revendication 6 ou la revendication 7, dans lequel :
un filtre (45) séparant le support d'échantillon d'essai (60) de la section d'imagerie (51) est prévu dans la région de mesure (91) ; et
la section d'analyse (250) est configurée pour, avant de détecter l'erreur de lumière parasite, détecter une erreur de filtre sur la base d'informations d'image du filtre (45) dans l'image de vérification capturée dans un état dans lequel le deuxième élément est éclairé par un illuminateur (52).

10. Système de mesure (10) selon l'une quelconque des revendications 1 à 9, dans lequel le deuxième élément inclut la section d'imagerie (51).

11. Système de mesure (10) selon l'une quelconque des revendications 1 à 9, dans lequel la section d'imagerie (51) et la section d'analyse (250) sont prévues au niveau d'un dispositif intelligent (50) qui est inclus dans le deuxième élément.

12. Système de mesure (10) selon l'une quelconque des revendications 1 à 9, dans lequel le deuxième élément inclut un boîtier (30) et un dispositif intelligent (50).
